Europäisches Patentamt

European Patent Office

Office européen des brevets

(19)

(11) Publication number: **0 243 947**
**A1**

## (12) EUROPEAN PATENT APPLICATION

(21) Application number: 87106225.3

(22) Date of filing: 29.04.87

(51) Int. Cl.4: **A61K 9/56**

(30) Priority: 30.04.86 DE 3614657

(43) Date of publication of application:
**04.11.87 Bulletin 87/45**

(84) Designated Contracting States:
**CH DE FR IT LI**

(71) Applicant: **DORNIER MEDIZINTECHNIK GMBH**
**Postfach 1128**
**D-8034 Germering 1(DE)**

(72) Inventor: **Schwarze, Werner, Dr.**
**Johanniter Strasse 4**
**D-7778 Stockach(DE)**

(74) Representative: **Landsmann, Ralf, Dipl.-Ing.**
**Kleeweg 3**
**D-7990 Friedrichshafen 1(DE)**

(54) Acoustic shock wave targeting of drug delivery in patients.

(57) Acoustic shock waves generated outside a patient's body are focused upon selected target zones within a patient's body to cause release of biologically active substances from liposomes administered to the patient. The procedures may serve to increase cell uptake of drugs and reduce systemic toxicity.

EP 0 243 947 A1

Fig.2

PROTEINE
POLARE KOPF-GRUPPEN
FETT-SÄURE-RESTE

## FIELD OF THE INVENTION

This invention relates to a controlled method for releasing pharmacologically active substances at a targeted location within the body in which acoustical shock waves are employed to release pharmacological agents from liposome carriers at the targeted area.

## BACKGROUND OF THE INVENTION

Acoustic shock wave treatments of patients have been known but not in relation to drug delivery systems. Shock wave treatments have been used widely to break up kidney stones without invasion of the patient's body by instruments. Such treatments are described, for example, in United States Patent No. 3,942,531 to Hoff et al. and in Extracorporeal Shock Wave Lithotripsy, 1982, edited by Christian Chaussy and published by Karger AC of Basel, Switzerland. The shock waves are generated exteriorly of the patient's body in a medium such as water and transmitted into the patient's body with suitable coupling that minimizes energy absorption at the interface with the patient's skin. As pointed out in Chaussy, the shock waves (which differ from ultrasound wave inputs in that they have a very steep compression pressure rise front and little or no tension component) may travel through normal body tissue at high pressure amplitudes without materially injuring such tissue.

Liposomes are closed bilayer membranes containing an entrapped aqueous phase. Liposomes may be any variety of unilamellar vesicles which possess a single membrane bilayer or multilamellar vesicles which are onion-like structures characterized by concentric membrane bilayers each separated from the next by a layer of water. The structure of a membrane bilayer is such that the hydrophobic (i.e., non-polar) "tails" of the lipid molecules orient toward the center of the bilayer while the hydrophilic (i.e. polar) "heads" orient towards the aqueous phases. Examples of lipids used in preparing liposomes include:
egg phosphatidylcholine (PC),
dilauroylphosphatidylcholine (DLPC),
dimyristoylphosphatidylcholine (DMPC),
dipalmitoylphosphatidylcholine (DPPC),
dioleoylphosphatidylcholine (COPC),
dimyristoylphosphatidylglycerol (CMPG),
dimyristoylphosphatidic acid (DMPA),
dipalmitoylphosphatidic acid (DPPA),
dipalmitoylphosphatidylethanolamine (DPPE),
brain phosphatidylserine (PS), and
brain sphingomyelin (SM).
Specific examples of some commonly used liposomes are set forth below.

| | | |
|---|---|---|
| PC:PS:C | 10:4:1 | PC:Phosphatidylcholine |
| PC:C:SA | 7:2:1; 10:4:3 | PS:Phosphatidyl serine |
| PC:C:DCP | 7:2:1 | C:Cholesterol |
| PS:PC:C | 3:7:10 | DCP:dicetylphosphate |

Application of liposomes to therapeutic uses is described in Liposomes: From Physical Structures to Therapeutic Applications, Knight, ed. Elsevier, North-Holland Biomedical Press, 1981. The possibilities of using these membrane vesicles for drug delivery systems has received considerable attention even though a number of problems have been noted. In a liposome drug delivery system the medicament, which may be soluble in water or in a non-polar solvent, is entrapped during liposome formation and then administered to the patient to be treated. See for example United States Patent Nos. 4,235,871 and 4,224,179.

Generally, biologically active substances such as antitumor agents, antimicrobial drugs, antiinflammatory and immunomodulatory agents and CNS-acting drugs have been incorporated into liposomes. These liposome encapsulated substances have generally been used for therapeutic applications such as cancer therapy, arthritis, metal chelation therapy, enzyme replacement therapy, hemophilia (factor VIII), myocardial infarction and the use of liposomes as radiopharmaceutical markers.

A problem area associated with liposome drug delivery systems has related to the release of the drugs in vivo. Release often has been accomplished by natural degradation of the liposomes in the presence of body fluid, even though there is much difficulty associated with this approach. A liposome which degrades too rapidly releases its contents uncontrollably in a short period of time. On the other hand, a liposome that is too stable also will be ineffective as a means for delivering drugs to the locations where they are desired.

Moreover, the methods which have been used for directing the liposomes to a particular target have not been entirely satisfactory. These methods include "passive" targeting methods which utilize natural localization patterns of liposomes as the result of such factors as liposome size and administration method. There also are "active" targeting methods which involve the alteration of the natural (passive) tissue disposition patterns of liposomes administered by different routes to cause interaction with specific cells, tissues, or organs. In addition, there are "physical targeting" methods which involve the construction of liposomes with physicochemical properties that result in drug release through lipid bilayer breakdown in response to exposure to specific environmental conditions such as changes in temperature and pH.

## SUMMARY OF THE INVENTION

It is an object of the present invention to provide for a liposomal delivery method which delivers and releases a biologically active substance to a particularly targeted area, thereby enhancing the uptake of the agent in a specifically targeted portion of the body.

It is a further object of the present invention to provide for a method for delivering an increased concentration of cytostatic drug to a targeted areas of the body while limiting the toxic effect of the drug and the rest of the body.

It is another object of the invention to enhance the cell uptake of a biologically active substance to a targeted area by directing acoustic shock waves to the targeted zone to concurrently release the substance from liposomes located in the targeted area and condition the cells in the target area.

Yet another object of the invention is to utilize acoustic shock waves to release drugs from liposomes formed from (1) lipid material which, if used alone, would provide lipid bilayers offering high resistance to leakage under the action of shock waves, and (b) another ingredient which coacts with the lipid material to provide lipid bilayers which are sensitive to shock waves.

Briefly stated, the present invention provides a selective delivery method comprising the steps of administering an encapsulated biologically active substance to a body and targeting the delivery of said substance to a particular location in said body, wherein said substance is encapsulated in a liposome upon administration and at least a portion of the substance is selectively released from the liposome by exposing the target area to acoustic shock waves.

The present invention is particularly advantageous in that it provides for enhanced control over the release of biologically active substances. That is, by means of focused acoustic shock wave exposure of a target volume, an encapsulated biologically active substance can be released in controlled doses at predetermined locations. Since the present invention provides for the release of a controlled amount of encapsulated substances at higher local concentration in the targeted area, this not only will enhance the probability but also the rate of cell uptake across the cell membrane of a therapeutic amount of the substance.

In addition to the improved targeting of the biologically active substance as described above, the shock waves can operate on cells in the target area to enhance treatment effectiveness. In the case of tumor treatment, for example, the shock waves used to release anti-tumor drug from the liposomes may injure cells in the tumor area so as to inhibit tumor growth and/or affect tumor cells so as to have an advantageous effect on the uptake of biologically active substances into such cells. The uptake by cells is characteristically due to diffusion processes which are countered by active membrane transport out of the cell. Shock waves can affect such systems to enhance the effect of a drug on the cells. It appears that shock wave treatment also can result in a greater exposure of lethal targets inside the cells, and/or repair mechanisms of the cell being compromised. Therefore, not only does the present invention enhance the uptake of the biologically active substance due to large local concentration of the drug in the target area. but it concurrently conditions the cells in the target area in such a manner that the drug uptake is enhanced.

3

With the foregoing and other objects, advantages, and features of the invention that will become hereinafter apparent, the nature of the invention may be more clearly understood by reference to the following detailed description of the invention, the attached drawings and the appended claims.

## BRIEF DESCRIPTION OF THE DRAWINGS

FIG. 1 provides a graphical comparison of features of a shock wave and an ultrasound wave.

FIG. 2 is a diagrammatic view for illustrating the nature of a lipid membrane.

FIG. 3 is a diagrammatic showing of a generally spherical vesicle formed from a lipid membrane.

FIG. 4 schematically illustrates a dialysis method for forming liposomes.

FIG. 5 schematically illustrates an apparatus set-up for generating shock waves outside a patient's body and using them to cause the release of biologically active substances from liposomes within the patient's body.

## DETAILED DESCRIPTION OF PREFERRED EMBODIMENTS

As generally described above, the present invention involves encapsulating a biologically active substance in a liposomal-type carrier, administering the encapsulated substance to the body, and subjecting a particular area of the body wherein it is desired to release the drug to shock wave treatment thereby releasing the substance in a particular targeted area.

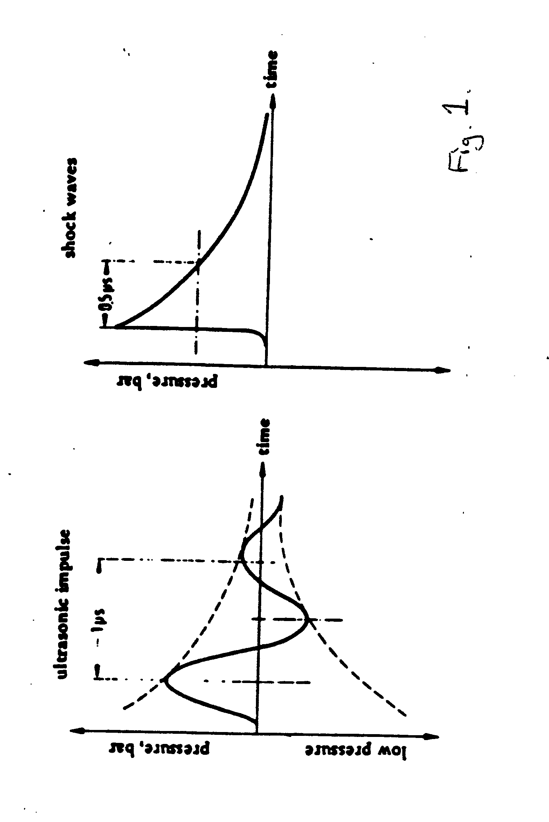

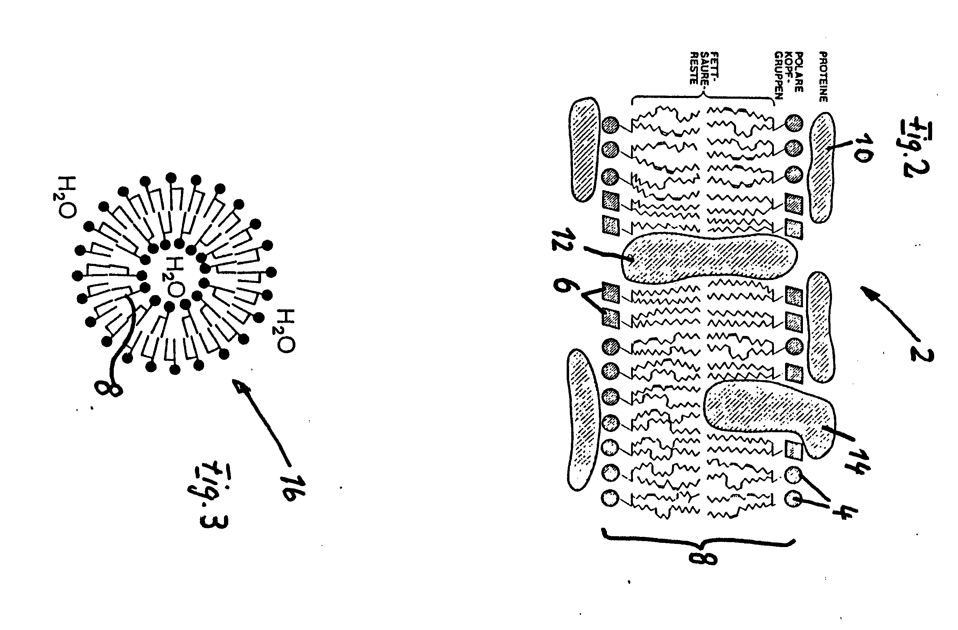

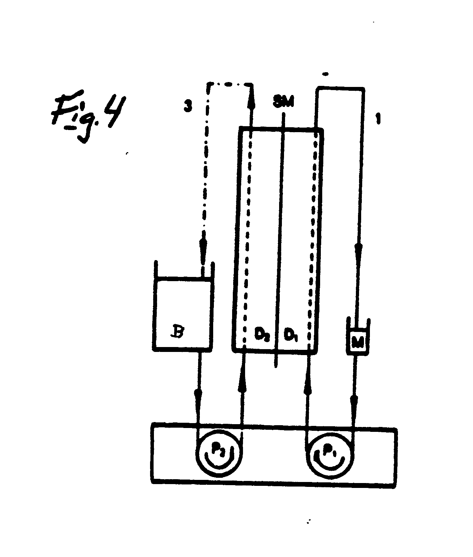

The nature of the shock waves will be explained with reference to Fig. 1 which depicts in idealized format characteristics of both an ultrasonic wave impulse which is not suitable for the practice of the present invention and an acoustic shock wave which is suitable. In each instance, the wave form is displayed in a pressure vs time diagram where pressure is indicated by the vertical direction and time is indicated by the horizontal direction. Pressures above ambient are located above the "time" axis and pressures below ambient are located below the "time" axis.

The ultrasound wave at the left of Fig. 1 is sinusoidal in nature in that the pressure rises regularly to a maximum value above ambient, falls regularly through ambient and on to a minimum value below ambient, and then rises regularly again. It will be understood that the portion of the curve above the "time" axis represent "compression" (shrinking) conditions in the medium in which the wave moves and the portion of the curve below the "time" axis represents "tension" (expanding) conditions in the medium.

The idealized shock wave at the right of Fig. 1 has a single pressure spike with very steep onset and a more gradual relaxation. Moreover, there is no "tension" component, in that pressures throughout are above ambient.

Some parameters associated with the shock waves which are employed in practicing the invention will promote a more complete understanding. With regard to the steep compression wave front, a typical rise time (period from onset to peak) is less than about $10^{-7}$ seconds. The maximum pressure attained will be quite high, appropriate values being in the range of about 10 bar to about 2000 bar, with values greater than 50 bar and preferably greater than 100 bar being favored in the absence of apparatus limitations or problems associated with the subject being treated. The overall duration of the whole wave event can be expected to be on the order of 5 times $10^{-6}$ seconds or less, and the wave will have little or no "tensile" (pressure below ambient) component. In this latter regard, any "tensile" component should be less than 25%, and preferably less than 10%, of the maximum pressure amplitude, and any oscillation should be minimized.

The absence of extensive tension wave components in shock waves is important from the standpoint of minimizing tissue damage in patients. Tension wave conditions are tolerated less well than compression wave conditions, and pressures far below ambient tend to cause damage to tissue. Hence, these components should be minimised both in amplitude and extent where possible.

The frequencies represented in the two types of waves diagrammed in Fig. 1 also are significant with respect to the biological effects the waves produce. The shock waves are broadband waves which have an extensive low frequency range as compared with ultrasound waves. Since the higher frequencies are more readily absorbed by biological material, the ultrasound waves tend to produce heat and cellular degradation, whereas shock waves have far less effect on the patient's body.

The liposomes which are useful in the present invention include those having the two standard forms, namely multilamellar vesicles made up of plural lipid bilayers and unilamellar vesicles containing a single bilayer. The lipid molecules form bilayer membranes when they are mixed with water because the molecules have hydrophobic or water-insoluble tails and hydrophilic or "polar" heads. Two fatty acid chains each containing from 10 to 24 carbon atoms typically make up the hydrophobic tail of most naturally occurring lipids. As a result, when lipids are mixed with water, the hydrophobic tails are attracted to each other to exclude water and the hydrophilic heads bind to water thereby forming a bilayer in which the fatty acid tails point into the membrane's interior, and the polar head groups point outward.

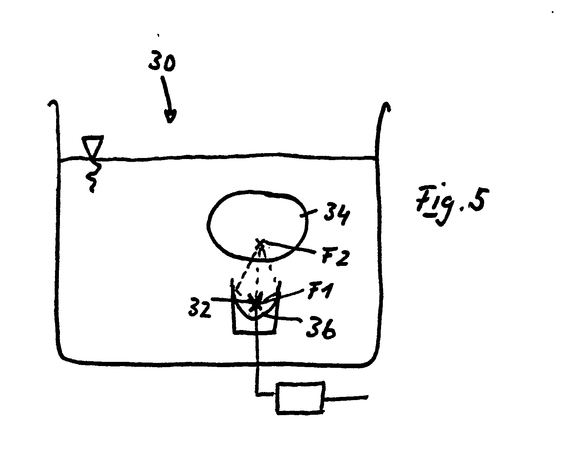

The structure of such membranes is generally characterized as a "fluid-mosaic-membrane" and is illustrated in Figure 2. Specifically, lipid molecules 2 having hydrophylic heads 4 and hydrophobic tails 6 form a double lipid layer 8 such that the hydrophobic tails face each other and the hydrophylic heads form the interface with the respective aqueous phases at the surface and interior of the liposome. For charged ions of the aqueous phases, the non-polar inner portion of the membrane formed by the hydrophobic tails represents a high energy barrier which is lipid-insoluble.

The lipid membranes are generally considered to be of a "liquid crystalline phase" having an order falling between the three dimensional order of a crystal and the random distribution of a liquid. Such liquid crystalline structures permits lateral diffusion (lateral interchange of sites within a layer) times on the order of $10^{-7}$ seconds. However, transverse exchange (i.e., flip-flop of lipid molecules from one layer to the other) is possible, but only in terms of hours or days. The resistance of artificial lipid membranes consisting of pure lipid is about $10^8 \Omega$-cm(ohm-centimeters) which is about $10^6$ times greater than that of cell membranes.

Figure 3 shows a typical structure of a spherical vesicle 16 which is formed by a single lipid bilayer 8 which closes upon itself. As discussed above, both multilamellar and unilamellar liposomes are useful in the present invention and are formed when the lipids are mixed with water. Unilamellar vesicles typically have diameters ranging from 200 to 1,000 angstrom units depending on the type of lipids used and the method of preparation. Larger vesicle diameters result from an increase in membrane thickness as the result of the use of long chain fatty acids. Multilamellar vesicles may be prepared having diameters are large as about $100\mu m$ (micrometers). For applications in which the liposomes are intended to circulate in a patient's bloodstream, liposome diameters of 1.5 micrometers or less (e.g., 1 micrometer) will be selected ordinarily to avoid stopping of the liposomes at unintended places in the blood paths.

The loading of biologically active substances into liposomes is well known. As the liposome forms, any water-soluble molecules that have been added to the water are incorporated into the aqueous spaces in the interior of the spheres, whereas any lipid-soluble molecules added to the solvent during vesicle formation are incorporated into the lipid bilayer. Specifically, the biologically active substances may be dissolved in an organic solvent together with the lipid and a detergent such as sodium cholate. In such a case, the biologically active substance is present in the lipid film before the film is solubilized. Alternatively, the substance may be dissolved in the aqueous phase (normally buffer) before the lipid film is dispersed.

There are several methods which have been used to form the vesicles from different lipids. These include ultrasonication of pure lipid, dialysis techniques, a method referred to in the art as the "freeze/thaw" method and swelling of lipid layers with water. Of these, the dialysis procedures are particularly effective.

There are several dialysis procedures which may be used to obtain a homogeneous population of single-shelled lipid vesicles with a diameter of about 90 (nm) nanometers. To prepare the lipid, chloroform is evaporated under $N_2$ in a round-bottom flask to yield a thin film on the glass wall. This procedure is repeated twice with ethyl ether. The lipid may the solubilized by adding a buffer containing 23 (mM) millimolar sodium cholate up to a concentration of 20 mg lipid per ml and rotating the flask for 20 minutes at room temperature and 2 minutes at 37°C. This is a stock solution and can be kept at -18°C for several months and up to years. To form the liposomes, the stock solution is diluted by a buffer containing 23 mM Na-cholate to a final lipid concentration of 10 mg/ml. The solution is transferred to 7 mm dialysis tubing and subjected to dialysis for 64 hours against buffer without cholate at 4°C.

Another dialysis procedure applies the use of active materials to bind the detergent removed from the liposomes. Similar to the first procedure, this method allows a reduction of the dialysis buffer by a factor of 100.

A third dialysis procedure which is particularly effective to produce liposomes uses a commercial available apparatus, the Liposomat ®. This method is schematically illustrated in Figure 4. Specifically, this method involves continuous and rapid detergent removal from the lipid-detergent micelle solutions and spontaneously leads to the formation of liposomes. The mixed lipid/detergent solution M is pumped by pump P1 through dialyser D1. The dialysis fluid B is continuously pumped by pump P2 through dialyser

5

D2. The detergent is continuously removed and the liposomes are formed within a few minutes. After one passage through the dialyser, the liposomes are already formed, still containing 50-60% residual detergent. Further cycles are used to remove more detergent. After about 90 minutes, the residual detergent concentration is below 1%. For this procedure a lipid concentration of 20 mg/ml is recommended.

The selection of the appropriate liposome and biologically active substance for delivery in vivo will depend on a number of factors including proposed use, route of administration, dose, properties of the substance, efficiency of substance entrapment, liposome size and composition, tissue disposition kinetics of clearance from the circulation if injected i.v.

For purposes of the present invention, it will at times be desirable to incorporate irregularities into the membrane to facilitate drug release in response to shock waves. Such irregularities may be formed by using a mixture of lipids having different length fatty acid chains or alternatively, by incorporating proteins into the membrane. The use of proteins 10, 12 and 14 in a membrane is illustrated in Fig. 2. These proteins may span or partially span the membrane or may be deposited thereon. It has been found that such irregularities allow for the use of liposomes having lipid bilayers which alone are typically very stable but as the result of the presence of the irregularities, release the biologically active substance when the liposome is subjected to shock waves.

To demonstrate the effect of incorporating irregularities such as proteins into a liposome, it will be helpful to compare the effect of shock waves on liposomes containing substantially no proteins with the effect of shock waves on liposomes having proteins incorporated therein.

Carboxy-Fluorescein (Eastman), a fluorescent dye, was dissolved in a buffer (135 mM NaCl, 10 mM Hepes) at a pH of greater than 10. The buffer was then adjusted to a pH of 7.2. The concentration of the dye was about 100 mM. Sodium cholate (1%) was dissolved into this buffer and mixed 1:1 with a lipid solution (20 mg/ml) of asolektin, a lipid isolated from soybeans, also containing 1% cholate. This mixture was transferred to a dialysis tubing (Serva) and subjected to dialysis. For this purpose the dialysis tubing, together with 3,3 gr Biobeads (Fluka) and the buffer (containing the dye) was stirred in a flask for at least 24 hours. The vesicles obtained by this method had a homogeneous size-distribution with a diameter of approximately 90 nm (+/- 5%).

After completion of the dialysis, the vesicles were separated from excess dye in the external medium by means of column chromatography using a Sephadex G-50 column (Pharmacia) with dimensions of 25 cm x 1 cm. According to size, the vesicles appeared in the exclusion-volume of the column after 5 minutes at standard pressure. After 15 minutes, the excess dye leaves the column. The vesicle solution was then diluted for use. The resulting vesicles showed no passive leakage upon storage for 14 days. The fluorescence of the vesicle solution was then measured both prior to and after the shock wave treatment. An increase of the fluorescence signal after the shock wave application would indicate the successful exchange of dye solution from the internal medium of the vesicle to the external medium. A number of shock waves between 1 and 1000 (at 25 kV) were applied to the vesicle suspension. There was, however, no change of the fluorescence signal observed. Therefore, the vesicles had remained stable with no exchange between the internal and external medium.

Lipid vesicles, into which a purified membrane protein, the Na,K-ATPase is reconstituted, were produced with reconstituted Na,K-ATPase and the fluorescent dye. Separation from the excess dye was achieved with the Sephadex G-50 column. The vesicle concentration was adjusted in the same way described above. After shock wave treatment of the vesicle solution an increase of the fluorescence signal was observed. This shows that, by reconstitution of a protein and the corresponding perturbation of the lipid matrix, there results a release of the intravesicular volume.

To determine if a particular liposome is suitable for release by shock wave treatment, an easy in vitro test may be used. In essence, such a test involves incorporating into a liposome a fluorescent dye such as Carboxy-Fluorescein (Eastman). The dye should be such that it exhibits little or no fluorescence at the internal pH of the liposome. The liposomes containing the dye are then suspended in a buffer having a pH in which the dye exhibits greater fluorescence. After incorporating the dye, the fluorescence of liposome suspension is measured, the liposomes are then subjected to shock wave treatment in vitro and subsequently, the suspension is measured again for fluorescence. If there in an increase in the fluorescence of the suspension, this indicates that the liposome released at least a portion of the encapsulated dye.

To determine the applicability of a particular biologically active substance or liposome to a particular cell line, another simple in vitro test may be used. Liposomes containing a biologically active substance may be layered on top of a cell line and then subjected to treatment with shock waves. After about 10 minutes, the liposomes may be washed out and the incubation cell growth analyzed to determine the continuous viability of the cells and how many cells are no longer viable.

To determine the amount of a particular biologically active substance which is delivered to a targeted location, a radioimaging method may be used. That is, a radioactive substance such as technetium 99 (6 hr. half-life) encapsulated in liposomes may be administered along with the liposome encapsulated drug. This may be accomplished either by encapsulating the radioactive substance along with the drug or by encapsulating the radioactive substance in liposomes which are separate from those which carry the drug. The presence of the radioactive substance can then be monitored using gamma camera imaging. The amount of radioactivity at the target will correlate with the amount of biologically active substance which is delivered to the target. From this information, the amount of encapsulated substance to be administered for a particular application can be optimized.

Liposomes may be introduced in vivo by a number of different routes of administration such as intravenously (iv), intraperitoneally (ip), intramuscularly (im), subcutaneously (sc), orally and topically. However, the particular route of administration may be closely related to the particular treatment goal. The method of administration preferred for many purposes of the present invention is intravenous administration.

The targeting method of the present invention is further enhanced when combined with those targeting methods discussed above previously used with liposomes, namely passive, active and physicochemical targeting methods.

The present invention may advantageously be applied in a number of different contexts. For example, the targeted release of the intravesicular volume of liposomes distributed in the blood is particularly applicable for the delivery of cytostatic substances in or near a target organ.

Adriamycin (doxorubicin), for example, is a cytostatic drug which is used in the treatment of a variety of different cancers such as carcinomas of the breast, ovary, endometrium, bladder, thyroid and in combination regimes for diffused lymphomas and Hodgkin's disease. It is also useful in acute leukemias, Ewing's sarcoma, osteogenic sarcoma, soft tissue sarcoma and neuroblastoma as well as multiple myeloma and adenocarcinomas of the stomach, prostate and testis. Generally, doxorubicin binds tightly to DNA by its ability to intercalate between base pairs and, therefore, is preferentially concentrated in nuclear structures. Intercalation results in inhibition, by steric hindrance, of DNA synthesis and DNA-dependent RNA synthesis and the production of single strand breaks in DNA.

As is common with this type of drug, the use of Adriamycin results in several adverse side effects. The most important toxicities involve the heart and bone marrow. It may cause transient cardiac arrythmias and depression of myocardial function. Chronic cardiotoxicity has been reported by 30% of patients who received total doses greater than about 13.6 mg/kg (550 mg/m$^2$). Clinically undetectable cardiac damage seems to occur with each dose. Congestive heart failure may occur at low total doses or in several months after discontinuation of treatment. The use of this drug also results in leukopenia, thrombocytopenia, alopecia, nausea, vomiting, stomatitus and esophagitis. In addition, impairment of hepatic function may result which may lead to severe myelosuppressive toxicity with therapeutic dosages.

Cytostatic drugs generally experience a rapid uptake by all tissues. Therefore, it is difficult to supply enough of the drugs to a tumor without exposing the rest of the body to the toxic effects of the drugs. It is estimated that when cytostatic drugs such as Adriamycin are administered to the body in an untargeted manner, seventy-five percent of the drug is washed from the body, twenty-five percent is flooded throughout the body with only one percent actually treating the tumor.

The method of the present invention can reduce many of the above-described side effects of cytostatic drugs. Specifically, the present invention would allow for the use of relatively stable liposomes thereby promoting isolation of the drug from the rest of the body and minimizing the uncontrollable release of the cytostatic drug throughout the body. In addition, the targeted release by shock waves of an increased concentration of the drug in the vicinity of a tumor would likely enhance the uptake of an effective amount of the drug in the tumor. Furthermore, the dosage of the delivered drug may be controlled by regulating the number and timing of shock waves which are applied to a particular area of the body. This is especially the case when iv administration is used and the particular location of treatment is well supplied with blood ( e.g., the kidney or liver ).

Of course, there are other applications of the present invention which are available. For example, the method may be used for the release of anesthetics in a controlled and locally targeted manner so as to provide local anesthesia. Also, substances which prevent hemorrhaging may be released from liposomes in the area of the kidney during the treatment of kidney stones with shock waves, so as to help reduce bleeding during treatment.

Local disintegration of clots by controlled and targeted release of an appropriate biologically active substance is even another potential use of the present invention. The present invention is also applicable to local hyperthermia by controlled and targeted release of substances which in subsequent chemical reactions with a positive thermal effect locally overheat the respective volume.

Fig. 5 shows a diagrammatic view of an apparatus setup for releasing pharmacological preparations within a patient's body by shock wave treatments. A shock wave source 32 and a patient 34 are positioned in a container 30 of water which preferably is degassed. This source 32 is to be understood as a spark gap device driven by an electrical condensor which releases its energy in a very short time. An arc arises between the electrodes which, in approximately 1 microsecond, vaporizes the water surrounding the arc's path, establishing a plasma-like state. The result is an explosion-like vaporisation of the water which produces a shock wave that spreads out in a circular fashion.

With this embodiment, the shock wave source is arranged in the focus F1 of an ellipsoid of revolution 36. Shock waves emanating from the shock wave source are concentrated in the focus F2 where the organ to be treated is located and where lipid vesicles also are located. Since the speed of sound in the water bath is about the same as the speed of sound in body tissue, the illustrated arrangement advantageously couples the shock waves into the patient's body with little reflection of energy at the body-water interface and with little likelihood of injury to the patient, all in a manner well known to those skilled in medical applications of shock waves.

The illustrated arrangement also permits safe relative movement between shock wave source and parts of the patient's body, so that the shock waves may be focused to the internal zones where the release of drugs from the vesicles is desired.

Other physical arrangements are of course possible. For example, instead of using a water bath for the patient, it has been proposed to interpose a suitable diaphragm or membrane between the patient's skin and a water body in which shock waves are generated. With such arrangements, a flexible bellows can be used for coupling so that relative movements can be accomplished for focusing the shock waves as required.

Other systems for generating shock waves may of course be used if desired. It is known, for instance, that laser beam energy focused at the focal point F1 can produce an acoustical shock wave emanating from that point.

Other energy inputs (e.g., electromechanical sources and piezoelectric generators) and other focusing techniques (e.g., hemispheres, lenticular focusing by a lens or lens system) also are possible. An array of piezoelectric devices arranged on a spherical segment or concave support, and suitably driven to provide shock wave outputs, can provide shock waves which converge or focus at a point or zone spaced away from the support; and such an arrangement has been proposed for introducing shock waves into a patient's body.

The release of pharmacological agents from vesicles as a result of the effects of the shock waves upon the leakage or encapsulating characteristics of the vesicles is not in all instances the only significant result flowing from the shock wave treatments. Shock waves also may have medically beneficial effects in conditioning patient cells in the target zone for more efficient uptake of the drug released from the liposomes and/or in acting upon the cells in a manner which is medically complimentary to the action of the drug.

The present invention is further illustrated by the following Example. However, the Example is not to be construed as in any way limiting the present invention.


EXAMPLE


Using the method disclosed in Onuma et al, Jpn. J. Conc. Res. 77, 1161-1167 (1986), Andriamycin (ADM) may be encapsulated in liposomes. Egg yolk phosphatidylcholine (40 micromol), cholesterol (20 micromol), dipalmitoylphosphatidic acid (4 micromoles) and 3-(2-Pyridyldithio)-diproprionyldipalmiotylphosphatidylethanolamine (0.6 micromol) may be dissolved in an organic solvent such as chloroform and methanol, with the organic solvent being subsequently removed by evaporation. One ml of ADM solution (20 mg/ml in saline buffer) may then be added to the dried film with multilamellar vesicles being formed by vortex dispersion. Small unilamellar vesicles may then be formed by probe sonication, and unencapsulated ADM removed by gel filtration on a Sephadex G-50 (Pharmacia) column.

The liposomes should then be suspended in a physiological saline buffer containing approximately 155 mmol NaCl at a pH of 7.2. A 50 ml sample containing approximately 100 mg ADM may then be prepared for intravenous bolus administration.

0 243 947

The liposome sample may then be administered intravenously to a patient having ovarian cancer over a period of 10-15 minutes. After completion of the bolus administration, the patient is subjected to shock wave treatment to release ADM in the area of the tumor. The shock waves are focused over about a 3 cm³ are which includes the location of the tumor, and applied every few seconds over a period of time suitable to release a tumor cell inhibiting sufficient amount of ADM as monitored by radioimaging.

The foregoing description of the preferred embodiments of the invention is intended as exemplary, and it will be appreciated that many modifications and variations of the present invention are possible in light of the above teachings and within the purview of the appended claims without departing from the spirit and intended scope of the invention.

**Claims**

1. A method for delivering an increased concentration of cytostatic substance to the site of cancer cells without concurrently exposing all parts of the patient's body to the action of a high concentration of the cytostatic substance, said method comprising encapsulating the substance in liposomes, administering the liposomes to the patient so as to deliver at least a portion of the liposomes to the site of the cancer cells, and directing acoustic shock waves to said site to release the cytostatic substance from the liposomes located at said site.

2. A method for treating a tumor which comprises encapsulating a cytostatic drug in liposomes, delivering the liposomes containing the encapsulated drug to the site of the tumor, and focusing shock waves on the site of the tumor to release cytostatic drug from the liposomes at the site of the tumor.

3. A method of enhancing the cell uptake of a biologically active substance, said method comprising encapsulating the substance in a liposome, delivering the encapsulated substance to the site of the cell, and subjecting the liposome and the cell to treatment with shock waves to condition the cell and to release at least a portion of the biologically active substance from the liposome at the site of the cell.

4. A method according to claim 3, wherein said biologically active substance is a cytostatic substance and wherein said cell is a cancer cell.

5. A method according to claim 4 wherein the liposomes are administered intravenously in an amount sufficient to provide a treatment effective concentration at the site of the cancer cells, and wherein said shock waves are focused on the zone where the cancer cells are located so as not to effect substantial release of cytostatic substance from the liposomes in the bloodstream as a whole outside said zone.

6. A method of limiting the systemic toxicity of a pharmacological active agent administered to a mammal, comprising introducing the agent into the body of the mammal within shock wave sensitive liposomes, and thereafter directing acoustic shock waves to a target zone within the body of the mammal to release the agent from the liposomes at the desired target zone.

7. A method according to claim 6, wherein said liposomes have diameters of less than about 1.5 micrometers and said introduction step is carried out by intravenous injection.

8. A method according to claim 6, including forming said liposomes from (1) lipid material which provides lipid bilayers offering high resistance to leakage, and (2) another ingredient which coacts with the lipid material to provide lipid bilayers sensitive to shock waves.

9. A method according to claim 8, wherein the ingredient coacting with the lipid material is protein.

10. A method of providing a predetermined dose of a pharmacological active agent to a target area in the body of a mammal which comprises administering the agent to the mammal within shock wave sensitive liposomes, and thereafter directing acoustic shock waves to a target zone within the body of the mammal to release the agent from the liposomes at the desired target zone.

11. A method according to claim 10 wherein the pharmacological active agent is a cytostatic drug and the target zone includes cancer cells.

12. A method according to claim 11 wherein the cell uptake of the agent is enhanced by the shock wave treatment.

13. A method according to claim 10, further comprising concurrently administering liposome encapsulated tracer substance in a known proportion to said agent and monitoring the concentration of the tracer substance at the target.

14. A method according to claim 10 wherein the liposomes are substantially biologically stable and contain irregularities in their lipid bilayers.

15. A method according to claim 14 wherein the irregularities are proteins.

16. A method according to claim 10 wherein the cell-uptake in the target area of the agent is enhanced by shock wave treatment.

9

ultrasonic impulse

shock waves

pressure, bar

low pressure

1 µs

0,5 µs

pressure, bar

time

time

Fig. 1.

0 243 947

_Fig. 2_

**2**

**10**

**14**

**4**

PROTEINE

POLARE
KOPF-
GRUPPEN

FETT-
SAURE-
RESTE

**8**

**12**

**6**

$H_2O$

**16**

_Fig. 3_

$H_2O$

**8**

$H_2O$

Fig.4

Fig. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | BIOLOGICAL ABSTRACT, vol. 75, 1983, abstract no. 50885; J.R. TRACKER et al.: "Delivery of anti-tumor drug to bladder cancer by use of phase transition liposame and hypathermia", & J. OF UROLOGY (USA) 1982, vol. 127, no. 6, pages 1211-1214<br><br>--- | 1-16 | A 61 K 9/56 |
| A | DE-A-2 747 378 (CHOAY)<br>* claims 3, 7-11, 18; page 7, lines 23-32; paGE (; LINES \| ´*<br><br>--- | 8 | |
| A | EP-A-0 036 277 (THE REGENTS OF UNIVERSITY OF CALIFORNIA)<br>* page 3, lines 5-30 *<br><br>--- | 14-16 | **TECHNICAL FIELDS SEARCHED (Int. Cl.4)** |
| A | FR-A-2 564 319 (INSERM ET CNRS)<br>* abstract; page 1, line 27-32; page 2, lines 1-15; claims 1-5 *<br><br>----- | 14-16 | A 61 K 9/00<br>A 61 B 17/00 |

The present search report has been drawn up for all claims

| Place of search | Date of completion of the search | Examiner |
|---|---|---|
| BERLIN | 01-07-1987 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82